# EUROPEAN PATENT SPECIFICATION

(11) **EP 3 782 619 B1**
(45) Date of publication and mention of the grant of the patent: **25.02.2026**
(21) Application number: 19807386.8
(22) Date of filing: 24.05.2019
(51) Int. Cl.: A61K 31/365, A61P 25/14

(54) **USE OF GINKGO BILOBA TERPENE LACTONE IN PREPARATION OF DRUGS FOR PREVENTION AND/OR TREATMENT OF TREMORS AND HEALTHCARE PRODUCTS**
VERWENDUNG VON GINKGO-BILOBA-TERPENLACTON ZUR HERSTELLUNG VON ARZNEIMITTELN ZUR VORBEUGUNG UND/ODER BEHANDLUNG VON TREMOR UND GESUNDHEITSPRODUKTEN
UTILISATION DE TERPÈNE LACTONE DE GINKGO BILOBA DANS LA PRÉPARATION DE MÉDICAMENTS POUR LA PRÉVENTION ET/OU LE TRAITEMENT DE TREMBLEMENTS ET DE PRODUITS DE SOINS DE SANTÉ

(30) Priority: 25.05.2018 CN 201810513686
(43) Date of publication of application: 24.02.2021
(73) Proprietor: Chengdu Baiyu Pharmaceutical Co., Ltd., Chengdu, Sichuan 611130 (CN)
(72) Inventor: SUN, Yi, Chengdu, Sichuan 611130 (CN); LI, Huiqin, Chengdu, Sichuan 611130 (CN); JIN, Zhendong, Chengdu, Sichuan 611130 (CN)
(74) Representative: Novagraaf Group
(86) International application number: PCT/CN2019/088359
(87) International publication number: WO 2019/223787

(56) References cited:
- WO-A1-2005/117924
- CN-A- 101 014 370
- CN-A- 101 095 674
- CN-A- 101 095 674
- CN-A- 103 880 857
- CN-A- 104 173 332
- CN-A- 104 306 371
- CN-A- 106 727 501
- LING-YUN LI , XI-LIN ZHAO , XI-FENG FEI , ZHEN-LUN GU , ZHENG-HONG QIN , ZHONG-QIN LIANG : "Bilobalide inhibits 6-OHDA-induced activation of NF-kB and loss of dopaminergic neurons in rat substantia nigra", ACTA PHARMACOL SINICA, vol. 29, no. 5, 1 May 2008 (2008-05-01), pages 539 - 547, XP055656311, ISSN: 1671-4083, DOI: 10.1111/j.1745-7254.2008.00787.x
- LUO, HUIQIONG: "Effect of Ginkgolide on D-Glu Content in Midbrain of Parkinson's Disease Mice", MASTER'S THESIS, 30 March 2014 (2014-03-30), pages 1 - 46, XP009524282

## Description

### TECHNICAL FIELD

The present invention relates to the field of medicine, in particular to the use of ginkgo biloba terpene lactone in the preparation of drugs for the prevention and/or treatment of tremors, and healthcare products.

### BACKGROUND

Modern research shows that the main active ingredients in ginkgo biloba are flavonoids and ginkgo biloba terpene lactone compounds. Ginkgo biloba flavonoids include flavonol glycosides, biflavones and catechin compounds. Ginkgo biloba terpene lactone compounds include ginkgolide A, ginkgolide B, ginkgolide C, ginkgolide J, ginkgolide M, ginkgolide K, ginkgolide L, ginkgolide N, ginkgolide P , ginkgolide Q and bilobalides. The research results show that the isolated ginkgo biloba terpene lactone compounds are similar in structure. The difference between Ginkgolides A, ginkgolides B, ginkgolides C and ginkgolides M lies in whether there is a hydroxyl functional group on carbon at position 1, 3 or 7 of the molecular skeleton. Ginkgolide L, ginkgolide K and ginkgolide N are obtained through olefination by removing one H₂O molecule from ginkgolide A, ginkgolide B and ginkgolide C respectively. Ginkgolide P and Ginkgolide Q are different from the above molecules in that the tert-butyl position is hydroxylated. In addition, in 1971, Major, Weinges and Nakanishi et al. determined the structure of the compound bilobalide, which also contains three lactone rings and one tert-butyl group, but only contains one full carbon ring.

Tremor, defined as rhythmic and unconscious muscle activity in any part of the body, is one of the most common and most widely affected movement disorders, which can cause limb dysfunction and affect the quality of life of patients. There are many factors and diseases that induce tremor, so it is difficult to clinically analyze and diagnose its etiology. At present, there are more than ten types of tremor with different pathogenesis and progression levels, including physiological tremor, essential tremor, Parkinson's disease (PD) tremor, dystonic tremor, orthostatic tremor, psychogenic tremor, childhood tremor, peripheral neuropathic tremor, drug-toxic tremor, position-specific tremor, palatal muscle tremor, nystagmus, cerebellar tremor, Holmes tremor or vascular tremor, etc.

Ginkgo biloba terpene lactone is an active ingredient which accounts for 6wt% of an extract from Ginkgo biloba. It is reported that Ginkgo biloba terpene lactone has anti-allergic effect, anti-inflammatory effect, anti-shock effect, protection effect against ischemic damage, and protection effect against organ transplant rejection (Xiaoju GUAN, Advance in studies on pharmacological activities of ginkgolides, Issue 3, Volume 22, 1995). Jiangping XU et al reported that ginkgolide can reduce the cerebral vascular resistance and increase cerebral blood flow in anesthetized dogs, but does not affect heart rate and blood pressure (Jiangping XU, et al., Effects of ginkgolide on cerebral blood flow in dogs, Journal of Chinese Integrative Medicine. January 15, 2005). There is no report about the use of ginkgolides in tremor.

### SUMMARY

In order to solve the problem of the lack of drugs for the prevention and/or treatment of tremor in the current medical field, the present invention provides use of one or more of a ginkgo biloba terpene lactone compound or a pharmaceutically acceptable salt, hydrate, or solvatethereof, or any crystal form, racemate of same, or a mixture of same as an active ingredient in the preparation of a drug or healthcare product for the prevention and/or treatment of tremors.

In one aspect, provided is use of one or more of a ginkgo biloba terpene lactone compound or a pharmaceutically acceptable salt, hydrate, or solvate thereof, or any crystal form, racemateof same, or a mixture of same as an active ingredient in the preparation of a drug or healthcare product for the prevention and/or treatment of tremors; wherein the ginkgo biloba terpene lactone compound is selected from a compound of formula (I), a compound of formula (II) or a compound of formula (III);
wherein the compound of formula (I) has a structure of: 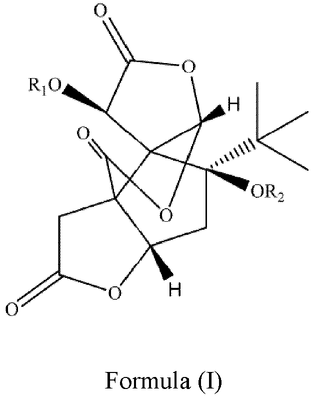
wherein, in the compound of formula (I), R₁ is selected from the group consisting of H, CH₃, CH₂CH₃, CH₂Ph, COCH₃ and SO₂CH₃, and R₂ is selected from the group consisting of H, CH₃, CH₂CH₃, CH₂Ph, COCH₃ and SO₂CH₃;
wherein the compound of formula (II) has a structure of: 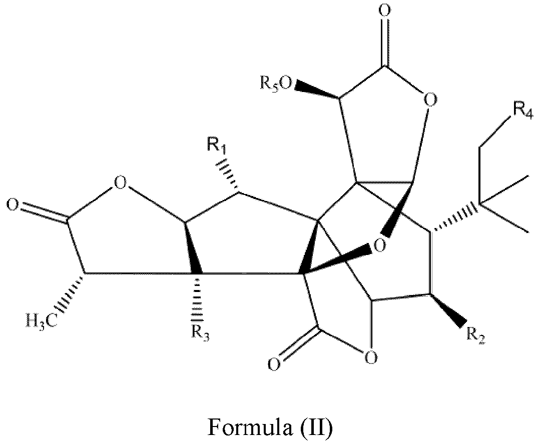
wherein, in the compound of formula (II), R₁ is selected from the group consisting of H and OH, R₂ is selected from the group consisting of OH and H, R₃ is selected from the group consisting of H and OH, R₄ is selected from the group consisting of OH and H, and R₅ is selected from the group consisting of H, CH₃, CH₂CH₃, CH₂Ph, COCH₃, SO₂CH₃, OH, H₂PO₃, H₂SO₃, -CH₂-Ar,- CH₂CH₂-Ar, -CH₂CH₂CH₂-Ar, -CONH-Ar, -CH₂O-Ar, -CH₂CH₂O-Ar, -CH₂CH₂CH₂O-Ar, -CO-Ar, -SO₂-Ar and -CO-A-Ar,
wherein A is C₂-C₈ alkenylene unsubstituted or substituted with C₁-C₆ alkyl, and wherein Ar is one or more selected from the group consisting of phenyl, pyridyl, pyrimidinyl, quinolyl or pyrazinyl, unsubstituted or substituted with 1-5 substituents selected from the group consisting of hydrogen, halogen, hydroxyl, cyano, carboxyl, C₁-C₆ alkyl, C₂-C₆ alkenyl, C₂-C₆ alkynyl, C₁-C₆ haloalkyl, C₁-C₆ alkoxy, C₁-C₆ acyloxy, C₁-C₆ ester group, C₁-C₁₀ alkyl, C₁-C₁₀ haloalkane, C₁-C₁₀ alkoxy, C₁-C₁₀ halogenated alkoxy, phenyl, phenoxy, aralkyl, aralkyloxy, -COR₆, -CONR₆R₇, -CO₂R₆, -CH₂OR₆, -NR₆R₇, -CH₂NR₆R₇, -CN and -NO₂; and
wherein R₆ is selected from the group consisting of hydrogen, C₁-C₁₀ alkyl, C₃-C₁₀ cycloalkyl, phenyl, pyridyl, pyrimidinyl, quinolyl and pyrazinyl, and R₇ is selected from the group consisting of hydrogen, C₁-C₁₀ alkyl, C₃-C₁₀ cycloalkyl, phenyl, pyridyl, pyrimidinyl, quinolyl and pyrazinyl; and
wherein the compound of formula (III) has a structure of: 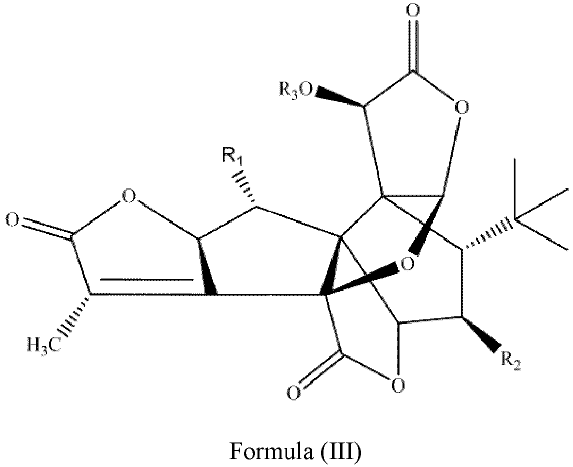
wherein, in the compound of formula (III), R₁ is selected from the group consisting of H and OH, R₂ is selected from the group consisting of OH and H, and R₃ is selected from the group consisting of H, CH₃, CH₂CH₃, CH₂Ph, COCH₃, SO₂CH₃, OH, H₂PO₃, H₂SO₃, -CH₂-Ar, -CH₂CH₂-Ar, -CH₂CH₂CH₂-Ar, -CONH-Ar, -CH₂O-Ar, -CH₂CH₂O-Ar, -CH₂CH₂CH₂O-Ar, -CO-Ar, -SO₂-Ar and -CO-A-Ar,
wherein A is C₂-C₈ alkenylene unsubstituted or substituted with C₁-C₆ alkyl, and Ar is one or more selected from the group consisting of phenyl, pyridyl, pyrimidinyl, quinolyl or pyrazinyl, unsubstituted or substituted with 1-5 substituents selected from the group consisting of hydrogen, halogen, hydroxyl, cyano, carboxyl, C₁-C₆ alkyl, C₂-C₆ alkenyl, C₂-C₆ alkynyl, C₁-C₆ haloalkyl, C₁-C₆ alkoxy, C₁-C₆ acyloxy, C₁-C₆ ester group, C₁-C₁₀ alkyl, C₁-C₁₀ haloalkane, C₁-C₁₀ alkoxy, C₁-C₁₀ halogenated alkoxy, phenyl, phenoxy, aralkyl, aralkyloxy, -COR₆, -CONR₆R₇, -CO₂R₆, -CH₂OR₆, -NR₆R₇, -CH₂NR₆R₇, -CN and -NO₂; and
wherein R₆ is selected from the group consisting of hydrogen, C₁-C₁₀ alkyl, C₃-C₁₀ cycloalkyl, phenyl, pyridyl, pyrimidinyl, quinolyl and pyrazinyl, and R₇ is selected from the group consisting of hydrogen, C₁-C₁₀ alkyl, C₃-C₁₀ cycloalkyl, phenyl, pyridyl, pyrimidinyl, quinolyl and pyrazinyl;
wherein the tremor is selected from the group consisting of physiological tremor, essential tremor, dystonic tremor, orthostatic tremor, psychogenic tremor, child tremor, peripheral neuropathic tremor, drug-toxic tremor, position-specific tremor, palatine muscle tremor, nystagmus, cerebellar tremor, Holmes tremor and vascular tremor.

Preferably, in the compound of formula (I), R₁ is H and R₂ is H;

Preferably, in the compound of formula (II), R₅ is selected from the group consisting of H, H₂PO₃, H₂SO₃, -CH₂-Ar, -CH₂CH₂-Ar, -CH₂CH₂CH₂-Ar, -CONH-Ar, -CH₂O-Ar, -CH₂CH₂O-Ar, -CH₂CH₂CH₂O-Ar, -CO-Ar and -SO₂-Ar, wherein Ar is selected from the group consisting of phenyl, pyridyl, pyrimidinyl and quinolyl, unsubstituted or substituted with 1-5 substituents selected from the group consisting of hydrogen, halogen, hydroxyl, C₁-C₁₀ alkyl, C₁-C₁₀ haloalkane, C₁-C₁₀ alkoxy, C₁-C₁₀ halogenated alkoxy, phenyl, phenoxy, aralkyl, aralkyloxy, -COR₆, -CONR₆R₇, -CO₂R₆, -CH₂OR₆, -NR₆R₇, -CH₂NR₆R₇, -CN and -NO₂; and wherein R₆ is selected from the group consisting of hydrogen, C₁-C₁₀ alkyl and C₃-C₁₀ cycloalkyl; and R₇ is selected from the group consisting of hydrogen, C₁-C₁₀ alkyl and C₃-C₁₀ cycloalkyl;

Preferably, in the compound of formula (III), R₃ is selected from the group consisting of H, -COAr and -CO-A-Ar, wherein Ar is selected from the group consisting of phenyl, pyridyl and pyrazinyl, unsubstituted or substituted with 1-5 substituents selected from the group consisting of halogen, hydroxyl, cyano, carboxyl, C₁-C₆ alkyl, C₂-C₆ alkenyl, C₂-C₆ alkynyl, C₁-C₆ haloalkyl, C₁-C₆ alkoxy, C₁-C₆ acyloxy, C₁-C₆ ester group, and wherein A is C₂-C₈ alkenylene unsubstituted or substituted with C₁-C₆ alkyl.

Preferably, the ginkgo biloba terpene lactone compound is selected from the group consisting of ginkgolide A, ginkgolide B, ginkgolide C, ginkgolide M, ginkgolide J, ginkgolide P, ginkgolide Q, ginkgolide K, ginkgolide L, ginkgolide N and bilobalide.

In a further aspect, the present invention provides a drug for the prevention and/or treatment of tremors, wherein the drug comprises an effective amount of ginkgo biloba terpene lactone and a pharmaceutically acceptable vehicle.

Preferably, the pharmaceutically acceptable vehicle comprises one or more selected from the group consisting of fillers, diluents, lubricants, glidants, anti-adherents, dispersants, humectants, adhesives, regulators, solubilizers, antioxidants, bacteriostat, emulsifiers, and disintegrants; wherein the adhesive comprises one or more selected from the group consisting of gum arabic, gelatin, sorbitol, tragacanth, cellulose, microcrystalline cellulose, sodium carboxymethyl cellulose, ethyl cellulose, hydroxypropyl methyl cellulose, syrup, starch syrup, and polyvinylpyrrolidone; wherein the filler comprises one or more selected from the group consisting of lactose, powdered sugar, dextrin, starch and derivatives thereof, cellulose and derivatives thereof, inorganic calcium salts, sorbitol, and glycine; wherein the lubricant comprises one or more selected from the group consisting of micronized silica gel, magnesium stearate, talc, aluminum hydroxide, boric acid, hydrogenated vegetable oil and polyethylene glycol; wherein the disintegrant comprises one or more selected from the group consisting of starch and derivatives thereof, polyvinylpyrrolidone, and microcrystalline cellulose; wherein the humectant comprises one or more selected from the group consisting of sodium dodecyl sulfate, water, and alcohol; wherein the antioxidant comprises one or more selected from the group consisting of sodium sulfite, sodium bisulfite, sodium metabisulfite, and dibutylbenzoic acid; wherein the regulator comprises one or more selected from the group consisting of hydrochloric acid, citric acid, potassium hydroxide, sodium citrate, and buffer; wherein the emulsifier comprises one or more selected from the group consisting of polysorbate-80, fatty acid sorbitan, Pluronic F-68, lecithin, and fabaceous lecithin; and wherein the solubilizer comprises one or more selected from the group consisting of Tween-80, bile, and glycerin.

Yet in a further aspect, the present invention provides a preparation comprising the above drug, wherein the preparation is in the forms of tablets, capsules, granules, pills, injections, needle injections, dripping pills, suspensions, powder injections, ointments, gel, aerosol or spray.

Yet in a further aspect, the present invention provides a healthcare product, wherein the healthcare product comprises an effective amount of ginkgo biloba terpene lactone and an excipient.

Preferably, the excipient comprises one or more selected from the group consisting of gum arabic, aspartame, benzoic acid, sodium benzoate, β-cyclodextrin, glacial acetic acid, erythrosin, erythrosine aluminum lake, erythritol, starch acetate, D-mannitol, dl-tartaric acid, sodium methyl p-hydroxybenzoate, ethyl p-hydroxybenzoate, sodium methyl p-hydroxybenzoate, mono- or di-glyceride fatty acid esters, indigo and aluminum lake thereof, titanium dioxide, beeswax, modified soybean phospholipids, glycerin, guar gum, silicon dioxide, pectin, potassium alginate, sodium alginate, fumaric acid, safflower yellow, monascus yellow pigment, talc powder, xanthan gum, methylcellulose, gellan gum, polydextrose, black bean red, polyoxyethylene sorbitan monooleate, polyglycol, cocoa shell color, L-tartaric acid, brilliant blue and aluminum lake thereof, dicalcium phosphate, tricalcium phosphate, caramel color, phospholipids, polyglycerol fatty acid esters, maltose, maltitol and maltitol liquid, roselle red, gelatin, xylitol, lemon yellow and aluminum lake thereof, citric acid, citric acid, potassium citrate, citric acid, pullulan, L-malic acid, DL-malic acid, pepper orange, capsicum red, grape skin red, disodium hydrogen phosphate, hydroxypropyl starch, hydroxypropyl distarch phosphate, hydroxypropyl methylcellulose, agar, carmine and aluminum lake thereof, sunset yellow and aluminum lake thereof, lactic acid, sucralose, sorbitol, sorbitol liquid, sodium carboxymethyl starch, sodium carboxymethyl cellulose, sodium carbonate, sodium bicarbonate, sodium saccharin, beet red, stevioside, sodium tripolyphosphate, natural amaranth, sorbic acid, potassium sorbate, stearic acid, magnesium stearate, acesulfame potassium, microcrystalline cellulose, starch sodium octenyl succinate, oxidized starch, oxidized hydroxypropyl starch, sodium copper chlorophyll, isomaltulose, acetylated distarch phosphate, allure red and aluminum lake thereof, gardenia blue, gardenia yellow, plant carbon black, edible essence, caprin, dextrin, starch, camellia seed oil, corn oil, sunflower oil, maltodextrin, corn starch, safflower seed oil, peanut oil, soybean oil, walnut oil, salad oil, potato starch, salt, water, condensed milk, vitamin C, vitamin E, amaranth and aluminum lake thereof, cocoa powder and cocoa butter, cream, wheat starch, olives oil, sesame oil, milk powder, rapeseed oil, polysorbate 80, pregelatinized starch, croscarmellose sodium, crospovidone, hydroxypropyl cellulose, povidone K30, low-substituted hydroxypropyl cellulose, simple syrup, black iron oxide, red iron oxide, coating premix, casein phosphopeptide, white sugar and white sugar products, red sugar, brown sugar, starch sugar, isomalt, lactitol, lactose, sucrose, glucose, lactose, fructose syrup, corn syrup, glucose syrup, yellow iron oxide, butylated hydroxyanisole, dibutylhydroxytoluene, sodium hexametaphosphate, medium chain triglycerides, ascorbyl palmitate, calcium silicate, rosemary extract, sodium starch octenyl succinate, sodium pyrophosphate, and calcium stearate.

Yet in a further aspect, the present invention provides a method for preventing and/or treating tremor, wherein the method comprises administering a therapeutically effective amount of ginkgo biloba terpene lactone as defined above to a subject in need.

Preferably, the tremor is selected from the group consisting of physiological tremor, essential tremor, dystonic tremor, orthostatic tremor, psychogenic tremor, child tremor, peripheral neuropathic tremor, drug-toxic tremor, position-specific tremor, palatine muscle tremor, nystagmus, cerebellar tremor, Holmes tremor and vascular tremor.

### Beneficial effects

The present invention provides use of ginkgo biloba terpene lactone in the preparation of a drug or healthcare product for the prevention and/or treatment of tremors, solving the problem of the lack of drugs for the prevention and/or treatment of tremor in the current medical field.

As shown in Experimental Example 1, the ginkgo biloba terpene lactone compound and composition provided in the present invention significantly improved the disease condition of the essential tremor model mice at each time point, and increased the mice's residence time on rod. In addition, the composition of ginkgolide B and bilobalide, and the composition of ginkgolide A, ginkgolide B and ginkgolide C provided by the present invention have an excellent effect in improving essential tremor model mice at different time points, and the effect is comparable to the effect of the positive drug proponol hydrochloride.

As shown in Experimental Examples 2-3, the present invention significantly improves the condition of patients with essential tremor or and patients with vascular tremor. The tremor score of the patients is reduced by more than 5 points, and no adverse reactions are found in clinical observation.

### DETAILED DESCRIPTION OF THE EMBODIMENTS

As described in the present invention, the term "prevention" refers to preventing the occurrence of a disease and/or preventing the recurrence of a disease.

Ginkgo biloba terpene lactone compounds used in the present invention are available commercially in the market, or can be prepared by existing separation and purification methods. After detection, all ginkgo biloba terpene lactone compounds should conform to the structure of the corresponding reference substance, and the purity detected by HPLC is above 99%.

When the ginkgo biloba terpene lactone of the present invention comprises two or more ginkgo biloba terpene lactone compounds, it can be prepared by combining the corresponding ginkgo biloba terpene lactone compounds.

### Experimental Example 1

Effect of ginkgo biloba terpene lactone on the Mouse Model of Essential Tremor Induced by Harmaline

### 1. Materials and methods

### 1.1 Experimental animals

The following animals are used: 100 Philadelphia Cancer Institute (ICR) male mice of SPF grade, weighted 18-22g, purchased from Chengdu Dashuo Experimental Animal Co., Ltd., with the animal production license number of SCXK (Sichuan) 2015-030.

### 1.2 Reagents and drugs

The following reagents and drugs are used: Ginkgolide A, ginkgolide B, ginkgolide C and bilobalide, provided by Chengdu Baiyu Pharmaceutical Co., Ltd.; Ginkgolide injection, provided by Chengdu Baiyu Pharmaceutical Co., Ltd., with National Medicine Standard Z20110035; Propranolol hydrochloride tablets, 10mg/tablet, provided by Jiangsu Yabang Aipusen Pharmaceutical Co., Ltd.,with National Medicine Standard H32020133; Ginaton^{®} Ginkgo Biloba Leave Extract Injection Solution, provided by Taiwan Chisheng Pharma & Biotech Co., Ltd., 5ml:17.5mg, with National Medicine Standard HC20140019; Harmaline, 25mg/bottle, purity >98% (HPLC), provided by Shanghai Macklin Biochemical Technology Co., Ltd.

### 1.3 Main instruments and equipment

The following equipment is used: Z600 Mouse Rota Rod for Fatigue Test, provided by Anhui Zhenghua Biologic Apparatus Facilities Co., Ltd.

### 1.4 Grouping and administration

100 mice were randomly assigned to ear label number and randomly divided into 10 groups according to weight stratified random number method, with 10 mice in each group.

The groups are: normal control group, model control group, propranolol hydrochloride tablet control group, ginkgo biloba leave extract injection solution control group, ginkgolide injection group, ginkgolide A group, ginkgolide B group, bilobalide group, ginkgolide B/bilobalide group (GB:BB=1:1w/w) and ginkgolide A/B/C group (A:B:C=1:1:1w/w/ w).

The normal control group and the model control group were administrated 0.9% sodium chloride injection by intraperitoneal injection at 0.1 mL/10 g·bw.

The hydrochloride tablet control group was administrated by gavage a solution (prepared with 0.9% sodium chloride injection) at 2 mg/kg·bw.

Ginkgolide injection group, ginkgolide A group, ginkgolide B group, bilobalide group, ginkgolide B/bilobalide group, ginkgolide A/B/C group were administrated by intraperitoneal injection at 5mg/kg·bw.

Ginkgo biloba leave extract injection solution control group was administered by intraperitoneal injection at 5 mg/kg·bw.

### 1.5 Rotating rod training

On the day before the rotating rod test, all mice were given rotating rod training, so that they were familiar with the relevant movements and were able to maintain balance by climbing continuously, without falling from the rotating rod at a low rotating speed (10 circles/min).

### 1.6 Rotating rod test

First, a rotating rod test was performed on all mice. The result was recorded as Pre-harmaline, used as the basic value of their exercise ability.

15 minutes after modeling, the time when it is observed that the mice given harmaline show obvious tremor, reduced activity and ataxia symptoms, was took as time 0. A rotating rod test was performed on all mice at time 0. Then the mice were administered immediately.

After time 0, a rotating rod test was performed on all mice at time 30 min (30min), time 60 min (60min), and time 90 min (90min).

Rotary rod test method: the mice were placed on the Rota Rod Fatigue Tester, and adapted for 20 seconds. After the mice stood steady on the rod, the tester was started and the rod was accelerated at a constant rate according to the setting. Each mouse's residence time on the rod, from the beginning of the test to the time when falling off the rod, was recorded respectively. The Rota Rod Fatigue Tester supports up to 6 channels for simultaneous testing.

The rod is set to rotate at an initial speed of 5 circles/min (5 c/min) which is increased at a constant rate to a maximum speed of 30 circles/min (30c/min) during 120 seconds, and kept at the maximum speed for 60 seconds. Therefore, each test costs 180 seconds in total, and the mice that had not yet fallen off the rod at the end of 180 seconds was recorded as having a residence time of 180 seconds on the rod.

Exercise ability change curves of the mice in each group were drawn, and the area under the curve was calculated.

### 1.6 Mathematical Statistics

The experimental data were processed by using a SPSS 19.0 statistical software. The measurement data are expressed as mean ± standard deviation (±SD). Data normality test was performed using the Kolmogorov-Smirnov method. If and the results conform to the normal distribution (P>0.05). one way ANOVA was adopted to compare the differences between groups, and if the results did not conform to the normal distribution (P≤0.05), Mann-Whitney U test (M-W method) was adopted to compare the differences between groups.

### 2. Experimental results

**Table 1 Effects of ginkgo biloba terpene lactone on the residence time on rod of the mice with essential tremor**

| **Group** | **Residence Time on Rod** | | | | |
|---|---|---|---|---|---|
| | **Pre-harmaline** | **Time 0** | **30 min** | **60 min** | **90 min** |
| Normal control group | 171.8±16.5 | 173.6±18.3 | 170.6±12.7 | 172.3±20.5 | 170.8±19.2 |
| Model control group | 170.8±18.5 | 43.8±14.1** | 20.5±10.5** | 21.3±13.7** | 31.7±15.2** |
| Propranolol hydrochloride tablet control group | 176.6±12.9 | 41.6±12.8** | 60.1±12.8^{##} | 77.6±10.3^{##} | 119.3±13.0^{##} |
| Ginkgo biloba leave extract injection solution control group | 175.1±20.4 | 38.8±11.8** | 23.5±11.2 | 25.3±11.7 | 36.2±13.6 |
| Ginkgolide injection group | 173.4±19.1 | 39.4±17.9** | 50.5±12.3^{##} | 62.6±9.9^{##} | 96.8±12.6^{##} |
| Ginkgolide A group | 170.6±21.2 | 42.6±10.8** | 50.8±10.9^{##} | 60.3±13.1^{##} | 91.4±11.5^{##} |
| Ginkgolide B group | 175.7+18.3 | 43.9±12.3** | 52.6±9.8^{##} | 65.8±12.8^{##} | 100.2±14.2^{##} |
| Bilobalide group | 171.6±17.9 | 40.0±15.6** | 48.2±12.8^{##} | 61.2±15.2^{##} | 90.6±13.9^{##} |
| Inkgolide B/ bilobalide group | 169.3±17.5 | 43.1±13.4** | 54.7±15.5^{##} | 68.3±14.1^{##} | 105.4±15.0^{##} |
| Ginkgolide A/B/C group | 173.2±22.9 | 42.7±12.7** | 57.6±20.1^{##} | 70.5±14.9^{##} | 107.7±19.6^{##} |

| | | | | | |
|---|---|---|---|---|---|
| Note: The experimental results are expressed as mean ± standard deviation. ** p<0.01 vs normal control group; ^{#} p<0.05, ^{##} p<0.01 vs model control group. | | | | | |

According to Table 1, at Pre-harmaline, there was no statistical difference in the residence time on rod in the rotating rod test of each group of mice, indicating that there was no significant difference in the basic exercise ability of the mice in each group.

At time 0, compared with the normal control group, the residence time on rod of mice in other groups in the rotating rod test was significantly reduced, and the difference was statistically significant (p<0.01), indicating that harmaline induced mouse model of essential tremor was successfully established, and the mice in each group that were given the modeling operation showed symptoms such as essential tremor and motor dysfunction.

At time points of 30min, 60min, and 90min, in each group except the normal control group, the exercise ability of mice gradually recovered and the residence time on rod in the rotating rod test gradually increased. At each time point, in the propranolol hydrochloride tablet control group, ginkgolide injection group, ginkgolide A group, ginkgolide B group, bilobalide group, ginkgolide B/ bilobalide group and ginkgolide A/B/C group, the residence time on rod of mice in the rotating rod test was significantly higher than that of the model control group, and the difference was statistically significant (p<0.05). However, there is no significant difference in the residence time on rod between mice in the ginkgo biloba leave extract injection solution control group and the model control group.

Essential tremor (ET) is a common dyskinesia disease. The incidence of ET is about 5%, and can rise to 20% in the elderly population, which is higher than the incidence of Parkinson's disease. Clinically, ET is characterized by postural or action tremor of the distal end of the upper limbs, accompanied by head, orofacial, or voice tremor.

The first-line drugs for essential tremor include propranolol, arotinolol and primidone. The most classic first-line drug commonly used in clinical practice is propranolol, which is still an off-label drug. Therefore, it is very important to explore a drug for the prevention and/or treatment of essential tremor.

In the existing research on tremor, there were studies using ginkgo extracts and ginkgolides for treating PD tremor. Dopaminergic agents such as levodopa have a significant effect on treatment of PD tremor, but are basically ineffective for treating essential tremor, because the pathogenesis of PD tremor is completely different from the pathogenesis of essential tremor. Therefore, it is difficult to explore a drug for the prevention and/or treatment of essential tremor.

According to Table 1, it can be seen that the ginkgo biloba terpene lactone compound and the composition of ginkgo biloba terpene lactone provided by the present invention can effectively prevent and/or treat the disease of essential tremor in mice, and the composition of ginkgolides A/B/C and the composition of ginkgolide B/BB achieved effects comparable to the efficacy of propranolol, and had a significantly improved effect on the disease.

**Table 2 Area under the rod exercise ability recovery curve in essential tremor mice**

| **Groups** | Ratio of area under the curve of each group to area under the curve of the model control group |
|---|---|
| Normal control group | 6.47 |
| Model control group | 1 |
| Propranolol hydrochloride tablet control group | 2.74 |
| Ginkgo biloba leaves extract injection solution control | 1.08 |
| group | |
| Ginkgolide injection group | 2.28 |
| Ginkgolide A group | 2.24 |
| Ginkgolide B group | 2.39 |
| Bilobalide group | 2.20 |
| Inkgolide B/bilobalide group | 2.48 |
| Ginkgolide A/B/C group | 2.55 |

According to Table 2, in the propranolol hydrochloride tablet control group, ginkgolide injection group, ginkgolide A group, ginkgolide B group, bilobalide group, ginkgolide B/bilobalide group, and ginkgolide A/B/C group, the recovery of exercise ability of mice in the rotating rod test was significantly better than that of model control mice, and the difference was statistically significant (p<0.05). There is no significant difference in the recovery of exercise ability between mice in the ginkgo biloba leave extract injection solution control group and the model control group.

### Example 1 Ginkgo biloba terpene lactone dropping pills

Active ingredients comprise:
Bilobalide 1000g (1 part), Ginkgolide B 500g (0.5 parts),
Ginkgolide A 250g (0.25 parts), and Ginkgolide C 250g (0.25 parts).

Preparation method comprise the steps of: mixing well the above raw materials with 1 part of glyceryl monostearate, 1 part of polyethylene glycol, 1 part of carrageenan, 1 part of gum arabic, 1 part of hydroxypropyl methyl cellulose, etc., and placing the obtained mixture to a dropping pill machine equipped with a device adapted for carrying out dissolving with water, heating the mixture evenly under routine stirring to obtain a melt, and then dropping the melt into a cooling liquid which is immiscible with the melt, thus forming pills after cooling, taking out the pills and wiping off the cooling liquid adhering to the surface thereof, and drying the pills at low temperature.

### Example 2 Ginkgo biloba terpene lactone dropping pills

### Active ingredients comprise:

Bilobalide 1000g (1 part), and Ginkgolide B 1000g (1 part).

Preparation method comprise the steps of: mixing well the above raw materials with 1 part of glyceryl monostearate, 1 part of polyethylene glycol, 1 part of carrageenan, 1 part of gum arabic, 1 part of hydroxypropyl methyl cellulose, etc., and placing the obtained mixture to a dropping pill machine equipped with a device adapted for carrying out dissolving with water, heating the mixture evenly under routine stirring to obtain a melt, and then dropping the melt into a cooling liquid which is immiscible with the melt, thus forming pills after cooling, taking out the pills and wiping off the cooling liquid adhering to the surface thereof, and drying the pills at low temperature.

### Experiment 2 Observation on the effect of ginkgo biloba terpene lactone dripping pills on patients with essential tremor

### I. Diagnostic criteria

Core diagnostic criteria: A. Obvious and persistent postural and/or action tremor in the hands and forearms; B. No other neurological signs (except gear phenomenon and Froment sign); and C. can only show head tremor, but not accompanied by dystonia.

Supporting diagnostic criteria: A. Disease courses of more than 3 years; B. A positive family history; and C. A reduced tremor after drinking.

Exclusion criteria: A. There are factors that cause physiological hyperactive tremor; B. Those who was recently or is being administrated with tremor-causing drugs or are in the period of withdrawing the drugs; C. Those who have a history of neurological trauma 3 months before the onset; D. Those who have a history or clinical evidence of mental (psychological) tremor; and E. Those who have sudden onset or progressive deterioration of the condition.

### II. Scoring criteria

The following 6 items related to tremor are scored: 1) patient complaint; 2) upper limb tremor procedure; 3) head, jaw, tongue and lower limb tremor degree; 4) full water test; 5) dressing, eating, buttoning and chopstick using; and 6) drawing circles and straight lines. The score for each item is classified as: normal (scored 0 piont), mild (scored 1 point), moderate (scored 2 points) and severe (scored 3 points).

Adverse reactions: It is observed whether there are any adverse reactions in the gastrointestinal tract, heart rate and blood pressure, etc. before and after the treatment.

### Criterion of curative effect:

Significantly effective: the difference in tremor score before and after treatment is ≥ 7.
Effective: the difference in tremor score before and after the treatment is 4 to 6.
Slightly effective: the difference in tremor score before and after the treatment is 2 to 3.
Ineffective: the difference in tremor score before and after the treatment is -1 to 1.
Deterioration: the difference in tremor score before and after the treatment is ≤-2.

### III. General information of patients

Patient 1: female, 70 years old, with a disease course of 7 years, having main symptoms of head tremor and hands tremor, mainly characterized by rhythmic abduction tremor and handwriting deformation; accompanied with hypertension, taking antihypertensive drugs regularly, with normal blood lipids and blood sugar. The tremor of this patient is scored 14 points.

Patient 2: male, 81 years old, with a disease course of 3 years, having main symptoms of head tremor and hands tremor; accompanied with hypertension, taking antihypertensive drugs regularly, with normal blood lipids and blood sugar; Levodopa has no effect in this patient. The tremor of this patient is scored 12 pionts.

Patient 3: female, 82 years old, with a disease course of 3 years, having the main symptom of limbs tremor. The tremor of this patient is scored 10 points.

### IV. Treatment and effects

Patient 1 was administrated orally with ginkgo biloba terpene lactone dropping pills of example 1 at 90mg/day for 90 days. After the administration was completed, it was observed that the feet and hands tremor was significantly reduced, and the patient could participate in exercise and had a better spirit; and the tremor score was reduced to 6 points, showing the pills are significantly effective in treating tremor.

Patient 2 was administrated orally with ginkgo biloba terpene lactone dropping pills of example 1 at 90mg/day for 60 days. After the administration was completed, it was observed that the head and hands tremor was alleviated, the memory became better, and the external response was more positive; and the tremor score was reduced to 7 points, showing the pills are effective in treating tremor.

Patient 3 was administrated orally with ginkgo biloba terpene lactone dropping pills of example 1 at 90mg/day for 30 days. After the administration was completed, it was observed that the hands and feet tremor was significantly alleviated, and the spirit became better; and the tremor score was reduced to 6 points, showing the pills are effective in treating tremor.

### V. Adverse reactions and follow-up observation.

Adverse reactions: during the period of administrations, no adverse reactions were observed. Patients 1, 2 and 3 had stable blood pressure, normal heart rate and no gastrointestinal reaction.

Follow-up observation: patient 1 felt significantly relieved on 90 days after taking ginkgo biloba terpene lactone dropping pills at 90mg/day, so stopped the taking of the drug on her own. 30 days after stop taking the drugs, the tremor score was 11 points. This patient 1 took Ginaton ginkgo biloba leave extract tablet at 240mg/day on her own for 30 days, but head and hands tremor was not improved, and the tremor score is 12 points, showing Ginaton ginkgo biloba leave extract tablet is ineffective.

Patient 2 continued to take ginkgo biloba terpene lactone dropping pills at 30mg/day for 30 days, and the head and hands tremor was relieved continuously, and the tremor score was reduced to 5 points.

Patient 3 continued to take ginkgo biloba terpene lactone dropping pills at 30mg/day for 30 days, and the tremor score was reduced to 3 points.

### Experiment 3 Observation on the effect of ginkgo biloba terpene lactone dripping pills on patients with vascular tremor

### I. Diagnostic criteria

Diagnosis criteria: A. Reduced movement, muscle rigidity, forward gait, broken steps and reciprocating; B. Positive signs of the nervous system; C. A history of hypertension and cerebral arteriosclerosis.

Exclusion criteria: Tremor paralysis caused by drugs and other diseases are excluded.

### II. Evaluation criteria

The following 6 items related to tremor are scored: 1) patient complaint; 2) upper limbs tremor procedure; 3) head, jaw, tongue and lower limbs tremor degree; 4) full water test; 5) dressing, eating, buttoning and chopsticks using; 6) drawing circles and straight lines. The score for each item is classified as: normal (scored 0 piont), mild (scored 1 point), moderate (scored 2 points) and severe (scored 3 points).

Adverse reactions: It is observed whether there are any adverse reactions in the gastrointestinal tract, heart rate and blood pressure, etc. before and after the treatment.

### Criterion of curative effect:

Significantly effective: the difference in tremor score before and after treatment is ≥ 7.
Effective: the difference in tremor score before and after the treatment is 4 to 6.
Slightly effective: the difference in tremor score before and after the treatment is 2 to 3.
Ineffective: the difference in tremor score before and after the treatment is -1 to 1.
Deterioration: the difference in tremor score before and after the treatment is ≤-2.
Adverse reactions: It is observed whether there are any adverse reactions in the gastrointestinal tract, heart rate and blood pressure, etc. before and after the treatment.

### III. General information of patients

Patient 1: female, 56 years old, with a disease course of 2 years, a history of hypertension for 3 years, suffered from transient cerebral ischemia 3 years ago, presently having gear-like increase in extremities muscle tone, having no resting tremor, showing active side tendon reflex. Levodopa has no effect in this patient, and the tremor of this patient is scored 11 points.

Patient 2: male, 62 years old, with a disease course of 4 years, a history of heart disease for 5 years, presently having gear-like increase in extremities muscle tone, having no resting tremor, showing panic gait. Levodopa has no effect in this patient, and the tremor of this patient is scored 9 points.

Patient 3: male, 71 years old, with a disease course of 2 years, accompanied by hypertension and diabetes for 5 years, presently having gear-like increase in extremities muscle tone, showing slow movement. Levodopa has no effect in this patient. Head CT shows old cerebral infarction. The tremor of this patient is scored 12 points.

### IV. Treatment and effect

Patient 1 was administrated orally with ginkgo biloba terpene lactone dropping pills of example 2 at 90mg/day for 30 days. After the administration was completed, gear-like decrease in extremities muscle tone was observed, the tremor symptoms were alleviated, and the tremor score was reduced to 6 points, showing the pills are effective in treating tremor.

Patient 2 was administrated orally with ginkgo biloba terpene lactone dropping pills of example 2 at 90mg/day for 40 days. After the administration was completed, gear-like decrease in extremities muscle tone was observed, the gait was normal, the tremor symptoms basically disappeared, and the tremor score was reduced to 2 points, showing the pills are significantly effective in treating tremor.

Patient 3 was administrated orally with ginkgo biloba terpene lactone dropping pills of example 2 at 90mg/day for 20 days. After the administration was completed, gear-like decrease in extremities muscle tone was observed, the exercise was normal, the motivation to exercise increased, the tremor symptoms were alleviated, and the tremor score was reduced to 5 points, showing the pills are significantly effective in treating tremor.

### V. Adverse reactions

During the period of administrations, no adverse reactions were observed. Patients 1, 2 and 3 had stable blood pressure, normal heart rate and no gastrointestinal reaction.

## Claims

1. A drug or a healthcare product for use in the prevention and/or treatment of tremors, **characterized in that** the drug or the healthcare product comprises one or more of a ginkgo biloba terpene lactone compound or a pharmaceutically acceptable salt, hydrate, or solvate thereof, or any crystal form, racemate of same, or a mixture of same as an active ingredient; and the ginkgo biloba terpene lactone compound is selected from a compound of formula (I), a compound of formula (II) or a compound of formula (III);
wherein the compound of formula (I) has a structure of: 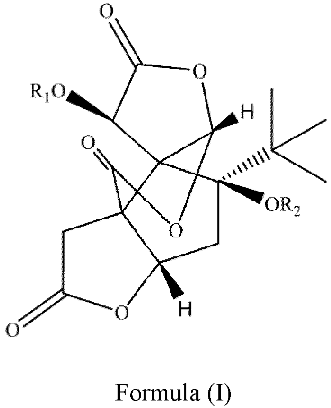
wherein, in the compound of formula (I), R₁ is selected from the group consisting of H, CH₃, CH₂CH₃, CH₂Ph, COCH₃ and SO₂CH₃, and R₂ is selected from the group consisting of H, CH₃, CH₂CH₃, CH₂Ph, COCH₃ and SO₂CH₃;
wherein the compound of formula (II) has a structure of: 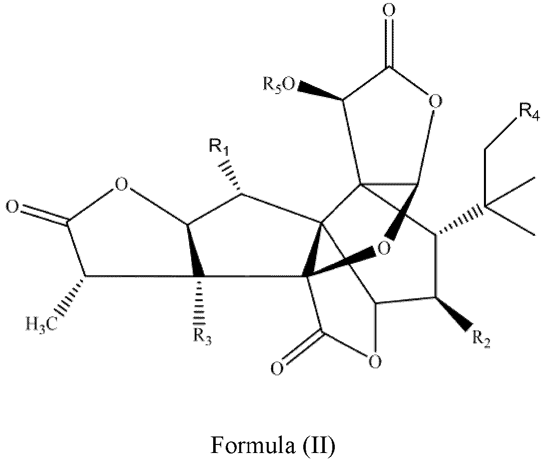
wherein, in the compound of formula (II), R₁ is selected from the group consisting of H and OH, R₂ is selected from the group consisting of OH and H, R₃ is selected from the group consisting of H and OH, R₄ is selected from the group consisting of OH and H, and R₅ is selected from the group consisting of H, CH₃, CH₂CH₃, CH₂Ph, COCH₃, SO₂CH₃, OH, H₂PO₃, H₂SO₃, -CH₂-Ar,- CH₂CH₂-Ar, -CH₂CH₂CH₂-Ar, -CONH-Ar, -CH₂O-Ar, -CH₂CH₂O-Ar, -CH₂CH₂CH₂O-Ar, -CO-Ar, -SO₂-Ar and -CO-A-Ar,
wherein A is C₂-C₈ alkenylene unsubstituted or substituted with C₁-C₆ alkyl, and wherein Ar is one or more selected from the group consisting of phenyl, pyridyl, pyrimidinyl, quinolyl or pyrazinyl, unsubstituted or substituted with 1-5 substituents selected from the group consisting of hydrogen, halogen, hydroxyl, cyano, carboxyl, C₁-C₆ alkyl, C₂-C₆ alkenyl, C₂-C₆ alkynyl, C₁-C₆ haloalkyl, C₁-C₆ alkoxy, C₁-C₆ acyloxy, C₁-C₆ ester group, C₁-C₁₀ alkyl, C₁-C₁₀ haloalkane, C₁-C₁₀ alkoxy, C₁-C₁₀ halogenated alkoxy, phenyl, phenoxy, aralkyl, aralkyloxy, -COR₆, -CONR₆R₇, -CO₂R₆, -CH₂OR₆, -NR₆R₇, -CH₂NR₆R₇, -CN and -NO₂; and
wherein R₆ is selected from the group consisting of hydrogen, C₁-C₁₀ alkyl, C₃-C₁₀ cycloalkyl, phenyl, pyridyl, pyrimidinyl, quinolyl and pyrazinyl, and R₇ is selected from the group consisting of hydrogen, C₁-C₁₀ alkyl, C₃-C₁₀ cycloalkyl, phenyl, pyridyl, pyrimidinyl, quinolyl and pyrazinyl; and
wherein the compound of formula (III) has a structure of: 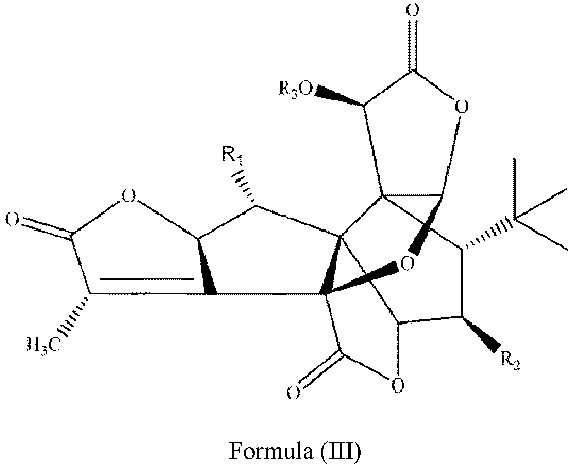
wherein, in the compound of formula (III), R₁ is selected from the group consisting of H and OH, R₂ is selected from the group consisting of OH and H, and R₃ is selected from the group consisting of H, CH₃, CH₂CH₃, CH₂Ph, COCH₃, SO₂CH₃, OH, H₂PO₃, H₂SO₃, -CH₂-Ar, -CH₂CH₂-Ar, -CH₂CH₂CH₂-Ar, -CONH-Ar, -CH₂O-Ar, -CH₂CH₂O-Ar, -CH₂CH₂CH₂O-Ar, -CO-Ar, -SO₂-Ar and -CO-A-Ar,
wherein A is C₂-C₈ alkenylene unsubstituted or substituted with C₁-C₆ alkyl, and Ar is one or more selected from the group consisting of phenyl, pyridyl, pyrimidinyl, quinolyl or pyrazinyl, unsubstituted or substituted with 1-5 substituents selected from the group consisting of hydrogen, halogen, hydroxyl, cyano, carboxyl, C₁-C₆ alkyl, C₂-C₆ alkenyl, C₂-C₆ alkynyl, C₁-C₆ haloalkyl, C₁-C₆ alkoxy, C₁-C₆ acyloxy, C₁-C₆ ester group, C₁-C₁₀ alkyl, C₁-C₁₀ haloalkane, C₁-C₁₀ alkoxy, C₁-C₁₀ halogenated alkoxy, phenyl, phenoxy, aralkyl, aralkyloxy, -COR₆, -CONR₆R₇, -CO₂R₆, -CH₂OR₆, -NR₆R₇, -CH₂NR₆R₇, -CN and -NO₂; and
wherein R₆ is selected from the group consisting of hydrogen, C₁-C₁₀ alkyl, C₃-C₁₀ cycloalkyl, phenyl, pyridyl, pyrimidinyl, quinolyl and pyrazinyl, and R₇ is selected from the group consisting of hydrogen, C₁-C₁₀ alkyl, C₃-C₁₀ cycloalkyl, phenyl, pyridyl, pyrimidinyl, quinolyl and pyrazinyl;
wherein the tremor is selected from the group consisting of physiological tremor, essential tremor, dystonic tremor, orthostatic tremor, psychogenic tremor, child tremor, peripheral neuropathic tremor, drug-toxic tremor, position-specific tremor, palatine muscle tremor, nystagmus, cerebellar tremor, Holmes tremor and vascular tremor.

2. The drug or the healthcare product for use according to claim 1, wherein
in the compound of formula (I), R₁ is H and R₂ is H;
in the compound of formula (II), R₅ is selected from the group consisting of H, H₂PO₃, H₂SO₃, -CH₂-Ar, -CH₂CH₂-Ar, -CH₂CH₂CH₂-Ar, -CONH-Ar, -CH₂O-Ar, -CH₂CH₂O-Ar, -CH₂CH₂CH₂O-Ar, -CO-Ar and -SO₂-Ar, wherein Ar is selected from the group consisting of phenyl, pyridyl, pyrimidinyl and quinolyl, unsubstituted or substituted with 1-5 substituents selected from the group consisting of hydrogen, halogen, hydroxyl, C₁-C₁₀ alkyl, C₁-C₁₀ haloalkane, C₁-C₁₀ alkoxy, C₁-C₁₀ halogenated alkoxy, phenyl, phenoxy, aralkyl, aralkyloxy, -COR₆, -CONR₆R₇, -CO₂R₆, -CH₂OR₆, -NR₆R₇, -CH₂NR₆R₇, -CN and -NO₂; and wherein R₆ is selected from the group consisting of hydrogen, C₁-C₁₀ alkyl and C₃-C₁₀ cycloalkyl; and R₇ is selected from the group consisting of hydrogen, C₁-C₁₀ alkyl and C₃-C₁₀ cycloalkyl; and
in the compound of formula (III), R₃ is selected from the group consisting of H, -COAr and -CO-A-Ar, wherein Ar is selected from the group consisting of phenyl, pyridyl and pyrazinyl, unsubstituted or substituted with 1-5 substituents selected from the group consisting of halogen, hydroxyl, cyano, carboxyl, C₁-C₆ alkyl, C₂-C₆ alkenyl, C₂-C₆ alkynyl, C₁-C₆ haloalkyl, C₁-C₆ alkoxy, C₁-C₆ acyloxy, C₁-C₆ ester group, and wherein A is C₂-C₈ alkenylene unsubstituted or substituted with C₁-C₆ alkyl.

3. The drug or the healthcare product for use according to claim 2, wherein the ginkgo biloba terpene lactone compound is selected from the group consisting of ginkgolide A, ginkgolide B, ginkgolide C, ginkgolide M, ginkgolide J, ginkgolide P, ginkgolide Q, ginkgolide K, ginkgolide L, ginkgolide N and bilobalide.

4. The drug or the healthcare product for use according to any one of claims 1 to 3, wherein the drug further comprises a pharmaceutically acceptable vehicle.

5. The drug or the healthcare product for use according to claim 4, wherein the pharmaceutically acceptable vehicle comprises one or more selected from the group consisting of fillers, diluents, lubricants, glidants, anti-adherents, dispersants, humectants, adhesives, regulators, solubilizers, antioxidants, bacteriostat, emulsifiers, and disintegrants; wherein the adhesive comprises one or more selected from the group consisting of gum arabic, gelatin, sorbitol, tragacanth, cellulose, microcrystalline cellulose, sodium carboxymethyl cellulose, ethyl cellulose, hydroxypropyl methyl cellulose, syrup, starch syrup, and polyvinylpyrrolidone; wherein the filler comprises one or more selected from the group consisting of lactose, powdered sugar, dextrin, starch and derivatives thereof, cellulose and derivatives thereof, inorganic calcium salts, sorbitol, and glycine; wherein the lubricant comprises one or more selected from the group consisting of micronized silica gel, magnesium stearate, talc, aluminum hydroxide, boric acid, hydrogenated vegetable oil and polyethylene glycol; wherein the disintegrant comprises one or more selected from the group consisting of starch and derivatives thereof, polyvinylpyrrolidone, and microcrystalline cellulose; wherein the humectant comprises one or more selected from the group consisting of sodium dodecyl sulfate, water, and alcohol; wherein the antioxidant comprises one or more selected from the group consisting of sodium sulfite, sodium bisulfite, sodium metabisulfite, and dibutylbenzoic acid; wherein the regulator comprises one or more selected from the group consisting of hydrochloric acid, citric acid, potassium hydroxide, sodium citrate, and buffer; wherein the emulsifier comprises one or more selected from the group consisting of polysorbate-80, fatty acid sorbitan, Pluronic F-68, lecithin, and fabaceous lecithin; and wherein the solubilizer comprises one or more selected from the group consisting of Tween-80, bile, and glycerin.

6. The drug or the healthcare product for use according to claim 4 or 5, wherein the drug is prepared into a preparation in the forms of tablets, capsules, granules, pills, injections, needle injections, dripping pills, suspensions, powder injections, ointments, gel, aerosol or spray.

7. The drug or the healthcare product for use according to any one of claims 1 to 3, wherein the healthcare product further comprises an excipient.

8. The drug or the healthcare product for use according to claim 7, wherein the excipient comprises one or more selected from the group consisting of gum arabic, aspartame, benzoic acid, sodium benzoate, β-cyclodextrin, glacial acetic acid, erythrosin, erythrosine aluminum lake, erythritol, starch acetate, D-mannitol, dl-tartaric acid, sodium methyl p-hydroxybenzoate, ethyl p-hydroxybenzoate, sodium methyl p-hydroxybenzoate, mono- or di-glyceride fatty acid esters, indigo and aluminum lake thereof, titanium dioxide, beeswax, modified soybean phospholipids, glycerin, guar gum, silicon dioxide, pectin, potassium alginate, sodium alginate, fumaric acid, safflower yellow, monascus yellow pigment, talc powder, xanthan gum, methylcellulose, gellan gum, polydextrose, black bean red, polyoxyethylene sorbitan monooleate, polyglycol, cocoa shell color, L-tartaric acid, brilliant blue and aluminum lake thereof, dicalcium phosphate, tricalcium phosphate, caramel color, phospholipids, polyglycerol fatty acid esters, maltose, maltitol and maltitol liquid, roselle red, gelatin, xylitol, lemon yellow and aluminum lake thereof, citric acid, citric acid, potassium citrate, citric acid, pullulan, L-malic acid, DL-malic acid, pepper orange, capsicum red, grape skin red, disodium hydrogen phosphate, hydroxypropyl starch, hydroxypropyl distarch phosphate, hydroxypropyl methylcellulose, agar, carmine and aluminum lake thereof, sunset yellow and aluminum lake thereof, lactic acid, sucralose, sorbitol, sorbitol liquid, sodium carboxymethyl starch, sodium carboxymethyl cellulose, sodium carbonate, sodium bicarbonate, sodium saccharin, beet red, stevioside, sodium tripolyphosphate, natural amaranth, sorbic acid, potassium sorbate, stearic acid, magnesium stearate, acesulfame potassium, microcrystalline cellulose, starch sodium octenyl succinate, oxidized starch, oxidized hydroxypropyl starch, sodium copper chlorophyll, isomaltulose, acetylated distarch phosphate, allure red and aluminum lake thereof, gardenia blue, gardenia yellow, plant carbon black, edible essence, caprin, dextrin, starch, camellia seed oil, corn oil, sunflower oil, maltodextrin, corn starch, safflower seed oil, peanut oil, soybean oil, walnut oil, salad oil, potato starch, salt, water, condensed milk, vitamin C, vitamin E, amaranth and aluminum lake thereof, cocoa powder and cocoa butter, cream, wheat starch, olives oil, sesame oil, milk powder, rapeseed oil, polysorbate 80, pregelatinized starch, croscarmellose sodium, crospovidone, hydroxypropyl cellulose, povidone K30, low-substituted hydroxypropyl cellulose, simple syrup, black iron oxide, red iron oxide, coating premix, casein phosphopeptide, white sugar and white sugar products, red sugar, brown sugar, starch sugar, isomalt, lactitol, lactose, sucrose, glucose, lactose, fructose syrup, corn syrup, glucose syrup, yellow iron oxide, butylated hydroxyanisole, dibutylhydroxytoluene, sodium hexametaphosphate, medium chain triglycerides, ascorbyl palmitate, calcium silicate, rosemary extract, sodium starch octenyl succinate, sodium pyrophosphate, and calcium stearate.

## Patentansprüche

1. Arzneimittel oder Gesundheitsprodukt zur Vorbeugung und/oder Behandlung von Tremor, **dadurch gekennzeichnet, dass** das Arzneimittel oder Gesundheitsprodukt eine oder mehrere von einer Ginkgo-biloba-Terpenlacton-Verbindung oder einem pharmazeutisch verträglichen Salz, Hydrat oder Solvat davon oder einer beliebigen Kristallform, einem Racemat davon oder eine Mischung davon als ein Wirkstoff umfasst; und die Ginkgo-biloba-Terpenlacton-Verbindung aus einer Verbindung von Formel (I), einer Verbindung von Formel (II) oder einer Verbindung von Formel (III) ausgewählt ist;
wobei die Verbindung von Formel (I) eine Struktur aufweist: 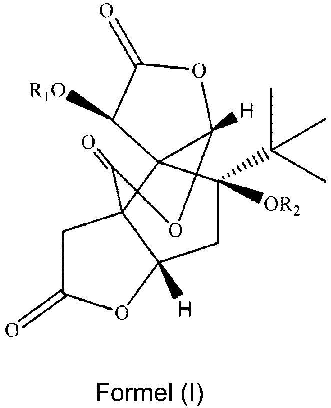
wobei, in der Verbindung von Formel (I), R₁ aus der Gruppe ausgewählt ist, bestehend aus H, CH₃, CH₂CH₃, CH₂Ph, COCH₃ und SO₂CH₃ und R₂ aus der Gruppe ausgewählt ist, bestehend aus H, CH₃, CH₂CH₃, CH₂Ph, COCH₃ und SO₂CH₃;
wobei die Verbindung von Formel (II) eine Struktur aufweist: 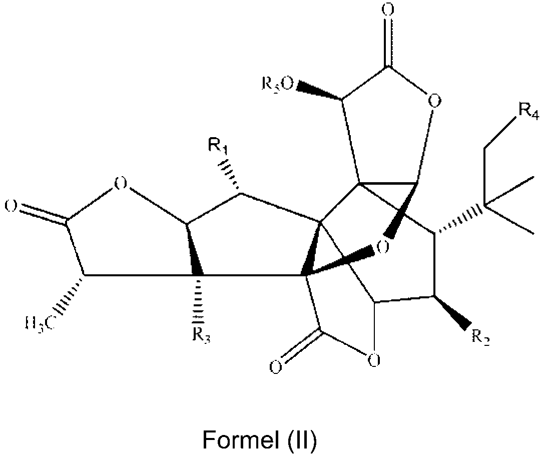
wobei, in der Verbindung von Formel (II), R₁ aus der Gruppe ausgewählt ist, bestehend aus H und OH, R₂ aus der Gruppe ausgewählt ist, bestehend aus OH und H, R₃ aus der Gruppe ausgewählt ist, bestehend aus H und OH, R₄ aus der Gruppe ausgewählt ist, bestehend aus OH und H, und R₅ aus der Gruppe ausgewählt ist, bestehend aus H, CH₃, CH₂CH₃, CH₂Ph, COCH₃, SO₂CH₃, OH, H₂PO₃, H₂SO₃, -CH₂-Ar,- CH₂CH₂-Ar, -CH₂CH₂CH₂-Ar, -CONH-Ar, -CH₂O-Ar, -CH₂CH₂O-Ar, -CH₂CH₂CH₂O-Ar, -CO-Ar, -SO₂-Ar und -CO-A-Ar,
wobei A C₂-C₈-Alkenylen ist, unsubstituiert oder substituiert mit C₁-C₆-Alkyl, und wobei Ar ein oder mehrere ist, die aus der Gruppe ausgewählt ist, bestehend aus Phenyl, Pyridyl, Pyrimidinyl, Chinolyl oder Pyrazinyl, unsubstituiert oder substituiert mit 1-5 Substituenten, die aus der Gruppe ausgewählt sind, bestehend aus Wasserstoff, Halogen, Hydroxyl, Cyano, Carboxyl, C₁-C₆-Alkyl, C₂-C₆-Alkenyl, C₂-C₆-Alkinyl, C₁-C₆-Halogenalkyl, C₁-C₆-Alkoxy, C₁-C₆-Acyloxy, C₁-C₆-Estergruppe, C₁-C₁₀-Alkyl, C₁-C₁₀-Halogenalkan, C₁-C₁₀-Alkoxy, halogeniertem C₁-C₁₀-Alkoxy, Phenyl, Phenoxy, Aralkyl, Aralkyloxy, -COR₆, -CONR₆R₇, -CO₂R₆, -CH₂OR₆, -NR₆R₇, -CH₂NR₆R₇, -CN und -NO₂; und
wobei R₆ aus der Gruppe ausgewählt ist, bestehend aus Wasserstoff, C₁-C₁₀-Alkyl, C₃-C₁₀-Cycloalkyl, Phenyl, Pyridyl, Pyrimidinyl, Chinolyl und Pyrazinyl, und R₇ aus der Gruppe ausgewählt ist, bestehend aus Wasserstoff, C₁-C₁₀-Alkyl, C₃-C₁₀-Cycloalkyl, Phenyl, Pyridyl, Pyrimidinyl, Chinolyl und Pyrazinyl; und
wobei die Verbindung von Formel (III) eine Struktur aufweist: 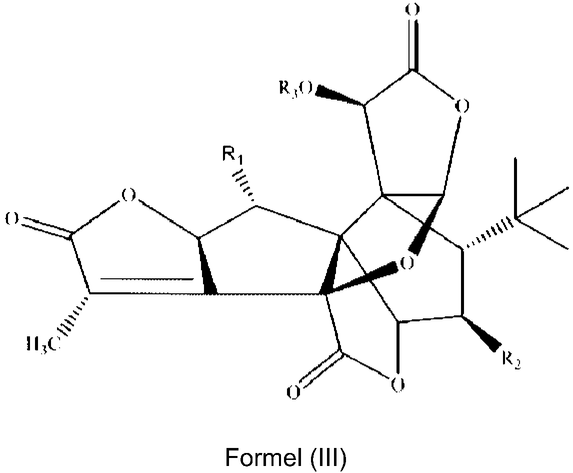
wobei, in der Verbindung von Formel (III), R₁ aus der Gruppe ausgewählt ist, bestehend aus H und OH, R₂ aus der Gruppe ausgewählt ist, bestehend aus OH und H, und R₃ aus der Gruppe ausgewählt ist, bestehend aus H, CH₃, CH₂CH₃, CH₂Ph, COCH₃, SO₂CH₃, OH, H₂PO₃, H₂SO₃, -CH₂-Ar, -CH₂CH₂-Ar, -CH₂CH₂CH₂-Ar, -CONH-Ar, -CH₂O-Ar, -CH₂CH₂O-Ar, -CH₂CH₂CH₂O-Ar, -CO-Ar, -SO₂-Ar und -CO-A-Ar,
wobei A C₂-C₈-Alkenylen ist, unsubstituiert oder substituiert mit C₁-C₆-Alkyl, und Ar ein oder mehrere ist, die aus der Gruppe ausgewählt sind, bestehend aus Phenyl, Pyridyl, Pyrimidinyl, Chinolyl oder Pyrazinyl, unsubstituiert oder substituiert mit 1-5 Substituenten, die aus der Gruppe ausgewählt sind, bestehend aus Wasserstoff, Halogen, Hydroxyl, Cyano, Carboxyl, C₁-C₆-Alkyl, C₂-C₆-Alkenyl, C₂-C₆-Alkinyl, C₁-C₆-Halogenalkyl, C₁-C₆-Alkoxy, C₁-C₆-Acyloxy, C₁-C₆-Estergruppe, C₁-C₁₀-Alkyl, C₁-C₁₀-Halogenalkan, C₁-C₁₀-Alkoxy, halogeniertem C₁-C₁₀-Alkoxy, Phenyl, Phenoxy, Aralkyl,
Aralkyloxy, -COR₆, -CONR₆R₇, -CO₂R₆, -CH₂OR₆, -NR₆R₇, -CH₂NR₆R₇, -CN und -NO₂; und
wobei R₆ aus der Gruppe ausgewählt ist, bestehend aus Wasserstoff, C₁- C₁₀-Alkyl, C₃-C₁₀-Cycloalkyl, Phenyl, Pyridyl, Pyrimidinyl, Chinolyl und Pyrazinyl, und R₇ aus der Gruppe ausgewählt ist bestehend aus Wasserstoff, C₁-C₁₀-Alkyl, C₃-C₁₀-Cycloalkyl, Phenyl, Pyridyl, Pyrimidinyl, Chinolyl und Pyrazinyl;
wobei der Tremor aus der Gruppe ausgewählt ist, bestehend aus physiologischem Tremor, essentiellem Tremor, dystonem Tremor, orthostatischem Tremor, psychogenem Tremor, Tremor bei Kindern, peripherem neuropathischem Tremor, medikamentenbedingtem Tremor, positionsspezifischem Tremor, Gaumenmuskeltremor, Nystagmus, zerebellärem Tremor, Holmes-Tremor und vaskulärem Tremor besteht.

2. Arzneimittel oder Gesundheitsprodukt zur Verwendung nach Anspruch 1, wobei
in der Verbindung von Formel (I), R₁ H ist und R₂ H ist;
in der Verbindung von Formel (II), R₅ aus der Gruppe ausgewählt ist, bestehend aus H, H₂PO₃, H₂SO₃, -CH₂-Ar, -CH₂CH₂-Ar, -CH₂CH₂CH₂-Ar, -CONH- Ar, -CH₂O-Ar, -CH₂CH₂O-Ar, -CH₂CH₂CH₂O-Ar, -CO-Ar und -SO₂-Ar, wobei Ar aus der Gruppe ausgewählt ist, bestehend aus Phenyl, Pyridyl, Pyrimidinyl und Chinolyl, unsubstituiert oder substituiert mit 1-5 Substituenten, die aus der Gruppe ausgewählt sind, bestehend aus Wasserstoff, Halogen, Hydroxyl, C₁-C₁₀-Alkyl, C₁-C₁₀-Halogenalkan, C₁-C₁₀-Alkoxy, C₁-C₁₀-halogeniertem Alkoxy, Phenyl, Phenoxy, Aralkyl, Aralkyloxy,-
COR₆, -CONR₆R₇, -CO₂R₆, -CH₂OR₆, -NR₆R₇, -CH₂NR₆R₇, -CN und -NO₂; und wobei R₆ aus der Gruppe ausgewählt ist, bestehend aus Wasserstoff, C₁-C₁₀-Alkyl und C₃-C₁₀-Cycloalkyl; und R₇ aus der Gruppe ausgewählt ist, bestehend aus Wasserstoff, C₁-C₁₀-Alkyl und C₃-C₁₀-Cycloalkyl; und
in der Verbindung von Formel (III), R₃ aus der Gruppe ausgewählt ist, bestehend aus H, -COAr und -CO-A-Ar, wobei Ar aus der Gruppe ausgewählt ist, bestehend aus Phenyl, Pyridyl und Pyrazinyl, unsubstituiert oder substituiert mit 1-5 Substituenten, die aus der Gruppe ausgewählt sind, bestehend aus Halogen, Hydroxyl, Cyano, Carboxyl, C₁-C₆-Alkyl, C₂-C₆-Alkenyl, C₂-C₆-Alkinyl, C₁-C₆-Halogenalkyl, C₁-C₆-Alkoxy, C₁-C₆-Acyloxy, C₁-C₆-Estergruppe, und wobei A C₂-C₈-Alkenylen ist, unsubstituiert oder substituiert mit C₁-C₆-Alkyl.

3. Arzneimittel oder Gesundheitsprodukt zur Verwendung nach Anspruch 2, wobei die Ginkgo-biloba-Terpenlacton-Verbindung aus der Gruppe ausgewählt ist, bestehend aus Ginkgolid A, Ginkgolid B, Ginkgolid C, Ginkgolid M, Ginkgolid J, Ginkgolid P, Ginkgolid Q, Ginkgolid K, Ginkgolid L, Ginkgolid N und Bilobalid.

4. Arzneimittel oder Gesundheitsprodukt zur Verwendung nach einem der Ansprüche 1 bis 3, wobei das Arzneimittel ferner eine pharmazeutisch verträgliche Trägersubstanz umfasst.

5. Arzneimittel oder Gesundheitsprodukt zur Verwendung nach Anspruch 4, wobei die pharmazeutisch verträgliche Trägersubstanz ein oder mehrere umfasst, die aus der Gruppe ausgewählt sind, bestehend aus Füllstoffen, Verdünnungsmitteln, Schmiermitteln, Flussregulierungsmitteln, Antihaftmitteln, Dispergiermitteln, Feuchthaltemitteln, Klebstoffen, Regulatoren, Lösungsvermittlern, Antioxidantien, Bakteriostatika, Emulgatoren und Zerfallsmittel; wobei der Klebstoff ein oder mehrere umfasst, die aus der Gruppe ausgewählt sind, bestehend aus Gummi arabicum, Gelatine, Sorbit, Tragant, Cellulose, mikrokristalliner Cellulose, Natriumcarboxymethylcellulose, Ethylcellulose, Hydroxypropylmethylcellulose, Sirup, Stärkesirup und Polyvinylpyrrolidon; wobei der Füllstoff eines oder mehrere umfasst, die aus der Gruppe ausgewählt sind, bestehend aus Lactose, Puderzucker, Dextrin, Stärke und Derivaten davon, Zellulose und Derivaten davon, anorganischen Calciumsalzen, Sorbit und Glycin; wobei das Schmiermittel eines oder mehrere umfasst, die aus der Gruppe ausgewählt sind, bestehend aus mikronisiertem Kieselgel, Magnesiumstearat, Talk, Aluminiumhydroxid, Borsäure, hydriertem Pflanzenöl und Polyethylenglycol; wobei das Zerfallsmittel eines oder mehrere umfasst, die aus der Gruppe ausgewählt sind, bestehend aus Stärke und Derivaten davon, Polyvinylpyrrolidon und mikrokristalliner Cellulose; wobei das Feuchthaltemittel eines oder mehrere umfasst, die aus der Gruppe ausgewählt sind, bestehend aus Natriumdodecylsulfat, Wasser und Alkohol; wobei das Antioxidationsmittel eines oder mehrere umfasst, die aus der Gruppe ausgewählt sind, bestehend aus Natriumsulfit, Natriumbisulfit, Natriummetabisulfit und Dibutylbenzoesäure; wobei der Regulator eines oder mehrere umfasst, die aus der Gruppe ausgewählt sind, bestehend aus Salzsäure, Citronensäure, Kaliumhydroxid, Natriumcitrat und Puffer; wobei der Emulgator eines oder mehrere umfasst, die aus der Gruppe ausgewählt sind, bestehend aus Polysorbat-80, Fettsäuresorbitan, Pluronic F-68, Lecithin und Hülsenfruchtlecithin; und wobei der Lösungsvermittler eines oder mehrere umfasst, die aus der Gruppe ausgewählt sind, bestehend aus Tween-80, Galle und Glycerin.

6. Arzneimittel oder Gesundheitsprodukt zur Verwendung nach Anspruch 4 oder 5, wobei das Arzneimittel in Form von Tabletten, Kapseln, Granulaten, Pillen, Injektionen, Nadelinjektionen, Tropfpillen, Suspensionen, Pulverinjektionen, Salben, Gel, Aerosol oder Spray zubereitet wird.

7. Arzneimittel oder Gesundheitsprodukt zur Verwendung nach einem der Ansprüche 1 bis 3, wobei das Gesundheitsprodukt ferner einen Arzneistoffträger umfasst.

8. Arzneimittel oder Gesundheitsprodukt zur Verwendung nach Anspruch 7, wobei der Arzneistoffträger ein oder mehrere umfasst, die aus der Gruppe ausgewählt sind, bestehend aus Gummi arabicum, Aspartam, Benzoesäure, Natriumbenzoat, β-Cyclodextrin, Eisessig, Erythrosin, Erythrosin-Aluminiumlack, Erythrit, Stärkeacetat, D-Mannit, DL-Weinsäure, Natriummethyl-p-hydroxybenzoat, Ethyl-p-hydroxybenzoat, Natriummethyl-p-hydroxybenzoat, Mono- oder Diglycerid-Fettsäureester, Indigo und Aluminiumlack davon, Titandioxid, Bienenwachs, modifizierter Sojabohnenphospholipide, Glycerin, Guarkernmehl, Siliciumdioxid, Pektin, Kaliumalginat, Natriumalginat, Fumarsäure, Saflorgelb, Monascusgelb-Pigment, Talkumpuder, Xanthangummi, Methylcellulose, Gellangummi, Polydextrose, Schwarzbohnenrot, Polyoxyethylensorbitanmonooleat, Polyglycol, Kakaoschalenfarbe, L-Weinsäure, Brillantblau und Aluminiumlack davon, Dicalciumphosphat, Tricalciumphosphat, Karamellfarbe, Phospholipide, Polyglycerinfettsäureester, Maltose, Maltit und Maltitflüssigkeit, Rosellerot, Gelatine, Xylit, Zitronengelb und Aluminiumlack davon, Citronensäure, Citronensäure, Kaliumcitrat, Citronensäure, Pullulan, L-Äpfelsäure, DL-Äpfelsäure, Pfefferorange, Capsicumrot, Traubenschalenrot, Dinatriumhydrogenphosphat, Hydroxypropylstärke, Hydroxypropyldistärkephosphat, Hydroxypropylmethylcellulose, Agar, Karmin und Aluminiumlack davon, Gelborange und Aluminiumlack davon, Milchsäure, Sucralose, Sorbit, Sorbitflüssigkeit, Natriumcarboxymethylstärke, Natriumcarboxymethylcellulose, Natriumcarbonat, Natriumbicarbonat, Natriumsaccharin, Rübenrot, Steviosid, Natriumtripolyphosphat, natürlichem Amaranth, Sorbinsäure, Kaliumsorbat, Stearinsäure, Magnesiumstearat, Acesulfamkalium, mikrokristalliner Cellulose, Stärkenatriumoctenylsuccinat, oxidierter Stärke, oxidierter Hydroxypropylstärke, Natriumkupferchlorophyll, Isomaltulose, acetylierte, Distärkephosphat, Allure Red und Aluminiumlack davon, Gardenia Blue, Gardenia Yellow, Pflanzenruß, essbarer Essenz, Caprin, Dextrin, Stärke, Kameliensamenöl, Maisöl, Sonnenblumenöl, Maltodextrin, Maisstärke, Distelsamenöl, Erdnussöl, Sojaöl, Walnussöl, Salatöl, Kartoffelstärke, Salz, Wasser, Kondensmilch, Vitamin C, Vitamin E, Amaranth und Aluminiumlack davon, Kakaopulver und Kakaobutter, Sahne, Weizenstärke, Olivenöl, Sesamöl, Milchpulver, Rapsöl, Polysorbat 80, vorverkleisterter Stärke, Croscarmellosenatrium, Crospovidon, Hydroxypropylcellulose, Povidon K30, niedrig substituierter Hydroxypropylcellulose, einfachem Sirup, schwarzem Eisenoxid, rotem Eisenoxid, Überzugsvormischung, Caseinphosphopeptid, weißem Zucker und Weißzuckerprodukten, rotem Zucker, braunem Zucker, Stärkezucker, Isomalt, Lactit, Lactose, Saccharose, Glucose, Lactose, Fructosesirup, Maissirup, Glucosesirup, gelbem Eisenoxid, Butylhydroxyanisol, Dibutylhydroxytoluol, Natriumhexametaphosphat, mittelkettiger Triglyceride, Ascorbylpalmitat, Calciumsilicat, Rosmarinextrakt, Natriumstärkeoctenylsuccinat, Natriumpyrophosphat und Calciumstearat.

## Revendications

1. Médicament ou produit de soins de santé pour utilisation dans la prévention et/ou le traitement de tremblements, **caractérisé en ce que** le médicament ou le produit de soins de santé comprend un ou plusieurs parmi un composé de terpène lactone de ginkgo biloba ou un sel, hydrate ou solvate pharmaceutiquement acceptable de celui-ci, ou l'une quelconque forme cristalline, l'un quelconque racémate de celui-ci, ou un mélange de celui-ci en guise d'ingrédient actif ; et le composé de terpène lactone de ginkgo biloba est choisi parmi un composé de formule (I), un composé de formule (II) ou un composé de formule (III) ;
dans lequel le composé de formule (I) a une structure de : 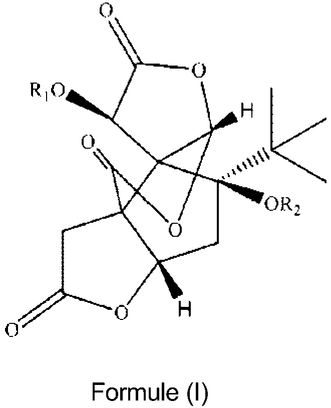
dans lequel, dans le composé de formule (I), R₁ est choisi dans le groupe constitué par H, CH₃, CH₂CH₃, CH₂Ph, COCH₃ et SO₂CH₃, et R₂ est choisi dans le groupe constitué par H, CH₃, CH₂CH₃, CH₂Ph, COCH₃ et SO₂CH₃ ;
dans lequel le composé de formule (II) a une structure de : 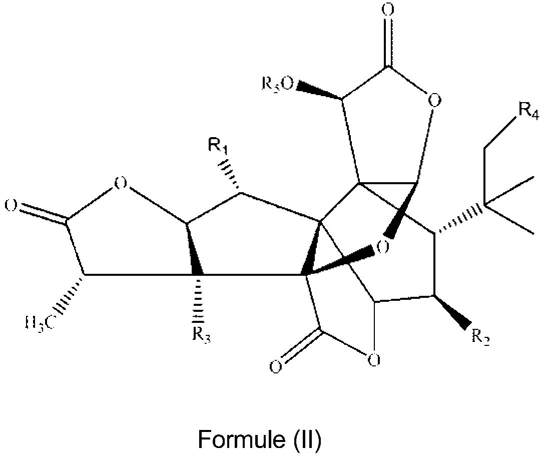
dans lequel, dans le composé de formule (II), R₁ est choisi dans le groupe constitué par H et OH, R₂ est choisi dans le groupe constitué par OH et H, R₃ est choisi dans le groupe constitué par H et OH, R₄ est choisi dans le groupe constitué par OH et H, et R₅ est choisi dans le groupe constitué par H, CH₃, CH₂CH₃, CH₂Ph, COCH₃, SO₂CH₃, OH, H₂PO₃, H₂SO₃, -CH₂-Ar,- CH₂CH₂-Ar, -CH₂CH₂CH₂-Ar, - CONH-Ar, -CH₂O-Ar, -CH₂CH₂O-Ar, -CH₂CH₂CH₂O-Ar, -CO-Ar, -SO₂-Ar et -CO-A-Ar,
dans lequel A est alcénylène en C₂ à C₈ non substitué ou substitué par alkyle en C₁ à C₆, et dans lequel Ar est un ou plusieurs choisis dans le groupe constitué par phényle, pyridyle, pyrimidinyle, quinolyle ou pyrazinyle, non substitué ou substitué par 1 à 5 substituants choisis dans le groupe constitué par hydrogène, halogène, hydroxyle, cyano, carboxyle, alkyle en C₁ à C₆, alcényle en C₂ à C₆, alcynyle en C₂ à C₆, haloalkyle en C₁ à C₆, alcoxy en C₁ à C₆, acyloxy en C₁ à C₆, groupe ester en C₁ à C₆, alkyl en C₁ à C₁₀, haloalcane en C₁ à C₁₀, alcoxy en C₁ à C₁₀, alcoxy halogéné en C₁ à C₁₀, phényle, phénoxy, aralkyle, aralkyloxy, -COR₆, -CONR₆R₇, -CO₂R₆, - CH₂OR₆, -NR₆R₇, -CH₂NR₆R₇, -CN et -NO₂ ; et
dans lequel R6 est choisi dans le groupe constitué par hydrogène, alkyle en C₁ à C₁₀, cycloalkyle en C₃ à C₁₀, phényle, pyridyle, pyrimidinyle, quinolyle et pyrazinyle, et R₇ est choisi dans le groupe constitué par hydrogène, alkyle en C₁ à C₁₀, cycloalkyle en C₃ à C₁₀, phényle, pyridyle, pyrimidinyle, quinolyle et pyrazinyle ; et
dans lequel le composé de formule (III) a une structure de : 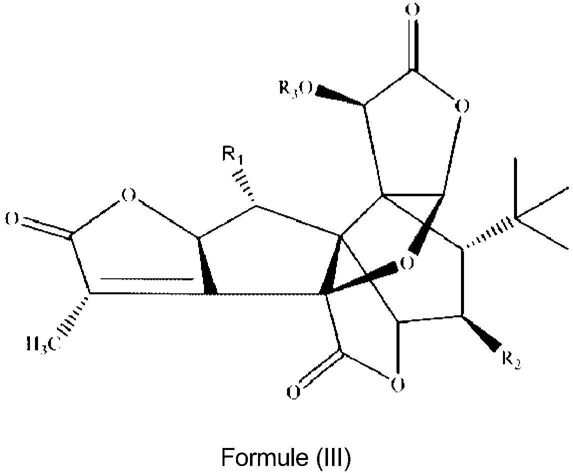
dans lequel, dans le composé de formule (III), R₁ est choisi dans le groupe constitué par H et OH, R₂ est choisi dans le groupe constitué par OH et H, et R₃ est choisi dans le groupe constitué par H, CH₃, CH₂CH₃, CH₂Ph, COCH₃, SO₂CH₃, OH, H₂PO₃, H₂SO₃, -CH₂-Ar, -CH₂CH₂-Ar, -CH₂CH₂CH₂-Ar, -CONH-Ar, -CH₂O-Ar, - CH₂CH₂O-Ar, -CH₂CH₂CH₂O-Ar, -CO-Ar, -SO₂-Ar et -CO-A-Ar,
dans lequel A est alcénylène en C₂ à C₈ non substitué ou substitué par alkyle en C₁ à C₆, et dans lequel Ar est un ou plusieurs choisis dans le groupe constitué par phényle, pyridyle, pyrimidinyle, quinolyle ou pyrazinyle, non substitué ou substitué par 1 à 5 substituants choisis dans le groupe constitué par hydrogène, halogène, hydroxyle, cyano, carboxyle, alkyle en C₁ à C₆, alcényle en C₂ à C₆, alcynyle en C₂ à C₆, haloalkyle en C₁ à C₆, alcoxy en C₁ à C₆, acyloxy en C₁ à C₆, groupe ester en C₁ à C₆, alkyl en C₁ à C₁₀, haloalcane en C₁ à C₁₀, alcoxy en C₁ à C₁₀, alcoxy halogéné en C₁ à C₁₀, phényle, phénoxy, aralkyle, aralkyloxy, -COR₆, -CONR₆R₇, -CO₂R₆, - CH₂OR₆, -NR₆R₇, -CH₂NR₆R₇, -CN et -NO₂ ; et
dans lequel R6 est choisi dans le groupe constitué par hydrogène, alkyle en C₁ à C₁₀, cycloalkyle en C₃ à C₁₀, phényle, pyridyle, pyrimidinyle, quinolyle et pyrazinyle, et R₇ est choisi dans le groupe constitué par hydrogène, alkyle en C₁ à C₁₀, cycloalkyle en C₃ à C₁₀, phényle, pyridyle, pyrimidinyle, quinolyle et pyrazinyle ;
dans lequel le tremblement est choisi dans le groupe constitué par tremblement physiologique, tremblement essentiel, tremblement dystonique, tremblement orthostatique, tremblement psychogène, tremblement infantile, tremblement neuropathique périphérique, tremblement toxique médicamenteux, tremblement spécifique à une position, tremblement des muscles palatins, nystagmus, tremblement cérébelleux, tremblement de Holmes et tremblement vasculaire.

2. Médicament ou produit de soins de santé pour utilisation selon la revendication 1, dans lequel
dans le composé de formule (I), R₁ est H et R₂ est H ;
dans le composé de formule (II), R5 est choisi dans le groupe constitué par H, H₂PO₃, H₂SO₃, -CH₂-Ar, -CH₂CH₂-Ar, -CH₂CH₂CH₂-Ar, -CONH-Ar, -CH₂O-Ar, - CH₂CH₂O-Ar, -CH₂CH₂CH₂O-Ar, -CO-Ar et -SO₂-Ar, dans lequel Ar est choisi dans le groupe constitué par phényle, pyridyle, pyrimidinyle et quinolyle, non substitué ou substitué par 1 à 5 substituants choisis dans le groupe constitué par hydrogène, halogène, hydroxyle, alkyle en C₁ à C₁₀, haloalcane en C₁ à C₁₀, alcoxy en C₁ à C₁₀, alcoxy halogéné en C₁ à C₁₀, phényle, phénoxy, aralkyle, aralkyloxy, -COR₆, - CONR₆R₇, -CO₂R₆, -CH₂OR₆, -NR₆R₇, -CH₂NR₆R₇, -CN et -NO₂ ; et dans lequel R₆ est choisi dans le groupe constitué par hydrogène, alkyle en C₁ à C₁₀ et cycloalkyle en C₃ à C₁₀ ; et R₇ est choisi dans le groupe constitué par hydrogène, alkyle en C₁ à C₁₀ et cycloalkyle en C₃ à C₁₀ ; et
dans le composé de formule (III), R₃ est choisi dans le groupe constitué par H, -COAr et -CO-A-Ar, dans lequel Ar est choisi dans le groupe constitué par phényle, pyridyle et pyrazinyle, non substitué ou substitué par 1 à 5 substituants choisis dans le groupe constitué par halogène, hydroxyle, cyano, carboxyle, alkyle en C₁ à C₆, alcényle en C₂ à C₆, alcynyle en C₂ à C₆, haloalkyle en C₁ à C₆, alcoxy en C₁ à C₆, acyloxy en C₁ à C₆, groupe ester en C₁ à C₆, et dans lequel A est alcénylène en C₂ à C₈ non substitué ou substitué par alkyle en C₁ à C₆.

3. Médicament ou produit de soins de santé pour utilisation selon la revendication 2, dans lequel le composé de terpène lactone de ginkgo biloba est choisi dans le groupe constitué par ginkgolide A, ginkgolide B, ginkgolide C, ginkgolide M, ginkgolide J, ginkgolide P, ginkgolide Q, ginkgolide K, ginkgolide L, ginkgolide N et bilobalide.

4. Médicament ou produit de soins de santé pour utilisation selon l'une quelconque des revendications 1 à 3, dans lequel le médicament comprend en outre un véhicule pharmaceutiquement acceptable.

5. Médicament ou produit de soins de santé pour utilisation selon la revendication 4, dans lequel le véhicule pharmaceutiquement acceptable comprend un ou plusieurs choisis dans le groupe constitué par charges, diluants, lubrifiants, agents glissants, anti-adhérents, dispersants, humectants, adhésifs, régulateurs, agents de solubilisation, antioxydants, agent bactériostatique, émulsifiants et agents délitants ; dans lequel l'adhésif comprend un ou plusieurs choisis dans le groupe constitué par gomme arabique, gélatine, sorbitol, gomme adragante, cellulose, cellulose microcristalline, carboxyméthylcellulose de sodium, éthylcellulose, hydroxypropylméthylcellulose, sirop, sirop d'amidon, et polyvinylpyrrolidone ; dans lequel la charge comprend un ou plusieurs choisis dans le groupe constitué par lactose, sucre en poudre, dextrine, amidon et dérivés de celui-ci, cellulose et dérivés de celle-ci, sels de calcium inorganiques, sorbitol et glycine ; dans lequel le lubrifiant comprend un ou plusieurs choisis dans le groupe constitué par gel de silice micronisé, stéarate de magnésium, talc, hydroxyde d'aluminium, acide borique, huile végétale hydrogénée et polyéthylène glycol ; dans lequel le délitant comprend un ou plusieurs choisis dans le groupe constitué d'amidon et dérivés de celui-ci, polyvinylpyrrolidone et cellulose microcristalline ; dans lequel l'humectant comprend un ou plusieurs choisis dans le groupe constitué par dodécylsulfate de sodium, eau et alcool ; dans lequel l'antioxydant comprend un ou plusieurs choisis dans le groupe constitué par sulfite de sodium, bisulfite de sodium, métabisulfite de sodium et acide dibutylbenzoïque ; dans lequel le régulateur comprend un ou plusieurs choisis dans le groupe constitué d'acide chlorhydrique, acide citrique, hydroxyde de potassium, citrate de sodium, et tampon ; dans lequel l'émulsifiant comprend un ou plusieurs choisis dans le groupe constitué par polysorbate-80, acide gras sorbitane, Pluronic F-68, lécithine et lécithine de fèves ; et dans lequel l'agent de solubilisation comprend un ou plusieurs choisis dans le groupe constitué par Tween-80, bile et glycérine.

6. Médicament ou produit de soins de santé pour utilisation selon la revendication 4 ou 5, dans lequel le médicament est préparé en une préparation sous les formes de comprimés, gélules, granulés, pilules, injections, injections à l'aiguille, pilules à effluves, suspensions, injections en poudre, pommades, gels, aérosols ou pulvérisation.

7. Médicament ou produit de soins de santé pour utilisation selon l'une quelconque des revendications 1 à 3, dans lequel le produit de soins de santé comprend en outre un excipient.

8. Médicament ou produit de soins de santé pour utilisation selon la revendication 7, dans lequel l'excipient comprend un ou plusieurs choisis dans le groupe constitué par gomme arabique, aspartame, acide benzoïque, benzoate de sodium, β-cyclodextrine, acide acétique glacial, érythrosine, laque d'aluminium d'érythrosine, érythritol, acétate d'amidon, D-mannitol, acide DL-tartrique, méthyl p-hydroxybenzoate de sodium, p-hydroxybenzoate d'éthyle, méthyl p-hydroxybenzoate de sodium, esters d'acides gras mono- ou diglycéridiques, indigo et laque d'aluminium de celui-ci, dioxyde de titane, cire d'abeille, phospholipides de soja modifiés, glycérine, gomme de guar, dioxyde de silicium, pectine, alginate de potassium, alginate de sodium, acide fumarique, jaune de carthame, pigment jaune de monascus, poudre de talc, gomme xanthane, méthylcellulose, gomme gellane, polydextrose, rouge de haricot noir, mono-oléate de polyoxyéthylène sorbitane, polyglycol, colorant de coque de cacao, acide L-tartrique, bleu brillant et laque d'aluminium de celui-ci, phosphate dicalcique, phosphate tricalcique, colorant caramel, phospholipides, esters d'acides gras de polyglycérol, maltose, maltitol et maltitol liquide, rouge de roselle, gélatine, xylitol, jaune citron et laque d'aluminium de celui-ci, acide citrique, citrate de potassium, acide citrique, pullulane, acide L-malique, acide DL-malique, orange poivré, rouge capsicum, rouge peau de raisin, hydrogénophosphate disodique, hydroxypropylamidon, phosphate d'hydroxypropylamidon, hydroxypropylméthylcellulose, agar-agar, carmin et laque d'aluminium de celui-ci, jaune soleil et laque d'aluminium de celui-ci, acide lactique, sucralose, sorbitol, sorbitol liquide, carboxyméthylamidon sodique, carboxyméthylcellulose sodique, carbonate de sodium, bicarbonate de sodium, saccharine sodique, rouge de betterave, stévioside, tripolyphosphate de sodium, amarante naturelle, acide sorbique, sorbate de potassium, acide stéarique, stéarate de magnésium, acésulfame de potassium, cellulose microcristalline, octényl succinate de sodium d'amidon, amidon oxydé, hydroxypropylate d'amidon oxydé, chlorophylle cuivrique sodique, isomaltulose, phosphate de di-amidon acétylé, rouge allure et laque d'aluminium de celui-ci, bleu gardénia, jaune gardénia, noir de carbone végétal, essence comestible, caprine, dextrine, amidon, huile de graines de camélia, huile de maïs, huile de tournesol, maltodextrine, amidon de maïs, huile de graines de carthame, huile d'arachide, huile de soja, huile de noix, huile de salade, amidon de pomme de terre, sel, eau, lait condensé, vitamine C, vitamine E, amarante et laque d'aluminium de celle-ci, poudre de cacao et beurre de cacao, crème, amidon de blé, huile d'olive, huile de sésame, lait en poudre, huile de colza, polysorbate 80, amidon prégélatinisé, croscarmellose sodique, crospovidone, hydroxypropylcellulose, povidone K30, hydroxypropylcellulose à faible substitution, sirop simple, oxyde de fer noir, oxyde de fer rouge, prémélange d'enrobage, phosphopeptide de caséine, sucre blanc et produits à base de sucre blanc, sucre rouge, sucre brun, sucre d'amidon, isomalt, lactitol, lactose, saccharose, glucose, lactose, sirop de fructose, sirop de maïs, sirop de glucose, oxyde de fer jaune, butylhydroxyanisole, dibutylhydroxytoluène, hexamétaphosphate de sodium, triglycérides à chaîne moyenne, palmitate d'ascorbyle, silicate de calcium, extrait de romarin, octényl succinate d'amidon sodique, pyrophosphate de sodium et stéarate de calcium.
